# EUROPÄISCHE PATENTSCHRIFT

(11) **EP 2 750 977 B1**
(45) Veröffentlichungstag und Bekanntmachung des Hinweises auf die Patenterteilung: **23.11.2016**
(21) Anmeldenummer: 12738076.4
(22) Anmeldetag: 11.07.2012
(51) Int. Cl.: B65B 55/10, A61L 2/08, A61L 2/10, A61L 2/18, A61L 2/22, B65B 55/04, B65B 55/08, B65D 25/00

(54) **VERFAHREN UND VORRICHTUNG ZUR KANTENENTKEIMUNG VON VERPACKUNGSMATERIAL**
METHOD AND DEVICE FOR DISINFECTING THE EDGES OF PACKAGING MATERIAL
PROCÉDÉ ET DISPOSITIF DE DÉSINFECTION DES ARÊTES D'UN MATÉRIAU D'EMBALLAGE

(30) Priorität: 31.08.2011 DE 102011111523
(43) Veröffentlichungstag der Anmeldung: 09.07.2014
(60) Teilanmeldung: 16183574.9
(73) Patentinhaber: SIG Technology AG, 8212 Neuhausen am Rheinfall (CH)
(72) Erfinder: FLÖRKE, Rudolf, 52428 Jülich (DE); GEISSLER, Hanno, 47802 Krefeld (DE); MAINZ, Hans-Willi, 52525 Heinsberg (DE)
(74) Vertreter: Cohausz & Florack
(86) Internationale Anmeldenummer: PCT/EP2012/063546
(87) Internationale Veröffentlichungsnummer: WO 2013/029856

(56) Entgegenhaltungen:
- EP-A1- 0 162 968
- EP-A1- 0 394 734
- GB-A- 1 513 266
- JP-A- 9 058 632
- JP-A- 2010 235 206
- US-A- 3 923 238
- US-A- 4 631 173

## Beschreibung

Die Erfindung betrifft ein Verfahren zur Behandlung von oben und/oder unten offenen Schnittkanten eines Rohlings (sog. "Packungsmantel", engl: ,sleeve') aus Verpackungsmaterial, insbesondere Papier/Kunststoff-Verbundmaterial, durch Aufbringen eines Behandlungsmittels sowie einen nach diesem Verfahren behandelten Packungsmantel.

Bei der Herstellung von Mehrschichtverbundpackungen, beispielsweise Getränkepackungen, werden unterschiedliche Verfahren angewandt.

Beispielsweise erfolgt die Herstellung von Packungen aus einzelnen Zuschnitten aus Papier/Kunststoff-Verbundmaterial. Hier werden zunächst aus einer Rolle an Verbundmaterial einzelne Zuschnitte gewonnen und diese anschließend mit einer Längsnaht versehen. Die Längsnähte werden durch Faltung und Versiegeln des Verbundmaterials derart erzeugt, dass ein in die Verpackung zu füllendes Produkt nicht mit einer offenen Kante des Verbundmaterials in Kontakt treten kann. Ein solcher Kontakt könnte zu einer Aufweichung des Verpackungsmaterials und zu einer Kontamination des abgefüllten Lebensmittels führen.

Die weitere Verarbeitung derart hergestellter Packungsmäntel, also das einseitige Verschließen auf der Ober- oder Unterseite der späteren Verpackung, das Entkeimen, das Befüllen und erneute Verschließen erfolgt meist direkt in der Füllmaschine.

Bei der Herstellung der Packung wird der Mantel zunächst an der Ober- oder Unterseite verschlossen. Anschließend wird das Packungsinnere gereinigt und gegebenenfalls desinfiziert, bevor der einseitig verschlossene Behälter der aseptischen Zone der Füllmaschine zugeführt wird. Dort erfolgt das Befüllen und Verschließen der Packung. Im Anschluss erfolgt die endgültige Formgebung der Packung. Ein solches Sterilisationsverfahren ist unter anderem in der DE 32 35 476 A1 beschrieben.

Unabhängig vom Herstellungsverfahren, erfolgt das Verschließen der Packung in der Regel durch Zusammenpressen und Versiegeln der Packstoffkanten beispielsweise mittels einer Sonotrode und einem Amboss. Es sind auch andere Verfahren zum Verschließen der Packung bekannt, beispielsweise elektromagnetische Induktion oder Heißluft in Verbindung mit mechanischem Verpressen.

Das Verschließen eines einseitig offenen, gefüllten Packungsmantels birgt das Risiko, dass insbesondere beim Versiegeln mit Ultraschall, aus den offenen Schnittkanten Staub aus dem Verpackungsmaterial geschleudert werden kann und dieser sowohl den aseptischen Bereich als auch die offenen Verpackungen verunreinigen kann.

Aus dem Stand der Technik sind verschiedene Verfahren zum Versiegeln der Kanten von Verpackungsrohlingen aus Karton bekannt. Das Versiegeln hat zum Ziel, das Eindringen von Flüssigkeit nach Fertigstellung der Verpackung in innen liegende Kanten der Verpackung zu verhindern.

Die gattungsgemäße DE 30 11 630 A1 offenbart ein Verfahren zur Behandlung von Verpackungsstirnseiten mit einem Öl, das in die Stirnseiten eindringt. Dabei dringt das Öl möglichst in alle Poren ein und kann durch Hitzeeinwirkung teilweise polymerisiert werden. Dadurch nimmt es an Viskosität zu.

Die WO 96/18544 A1 beschreibt eine Methode zum Versiegeln von Kanten von Verpackungen mit Wachs, Kunststoff, Klebstoff oder Schmelzkleber. Die Versiegelung wird dabei entweder durch eine Düse, durch Eintauchen oder Aufsprühen aufgetragen. Anschließend kann die Versiegelung durch Hitze ausgehärtet werden.

Aus der US 3 187 480 A ist es bekannt, einen Stapel von Kartonrohlingen zusammenzupressen und zum Schutz der Schnittkanten in ein heißes Bad aus Imprägnierlösung einzutauchen. Als Imprägnierlösung sind Glycin und Rizinusöl offenbart.

Es ist auch bereits aus der US 4 631 173 A bekannt, oben offene Behälter vor ihrer Befüllung mit einem Produkt mittels einem Sterilisationsmittel, welches als heißes Dampf-Gasgemisch in das Packungsinnere eingebracht wird, zu sterilisieren. Dabei wird das Dampf-Gasgemisch über die Packungsränder umgelenkt und kommt dabei mit den oben offenen Kanten des Behälters in Berührung. Das bekannte Verfahren dient daher zur Sterilisation der oben offenen Kanten der Behälter und zur Sterilisation des Behälterinneren.

Davon ausgehend liegt der Erfindung die Aufgabe zugrunde, ein einfaches und kostengünstiges Verfahren sowie einen Behälter zu entwickeln, bei denen das Risiko der Kontamination der Verpackungen, des Produktes und des aseptischen Bereichs einer Abfüllanlage beim Befüllen und Verschließen der Verpackungen reduziert wird.

Diese Aufgabe wird bei einem Verfahren nach dem Oberbegriff von Anspruch 1 dadurch gelöst, dass das Behandlungsmittel mindestens ein Entkeimungsmittel enthält, dass während des Aufbringens eine Mehrzahl von Packungsmänteln flachgefaltet zusammengefasst sind und dass das Entkeimungsmittel nach dem Aufbringen auf den Schnittkanten verbleibt und in das Verpackungsmaterial eindringt.

Der Mantel aus Verpackungsmaterial, auch Packungsmantel genannt, ist insbesondere zum Herstellen einer Verpackung für Nahrungsmittel, besonders flüssige Lebensmittel geeignet. Das Verpackungsmaterial ist bevorzugt Verbundverpackungsmaterial, insbesondere Papier/Kunststoff-Verbundverpackungsmaterial.

Bevorzugt sind die Packungsmäntel bei der Behandlung mittels dem erfindungsgemäßen Verfahren mit den oberen und/oder unteren Schnittkanten nach oben gerichtet. Das Entkeimungsmittel dringt so besonders gut in die oberste Kartonschicht des Verpackungsmaterials ein.

Bei der beispielhaften Herstellung von Papier/Kunststoff-Verbundverpackungsmaterial wird zunächst eine Trägerschicht, meist Papier oder Karton, beschichtet und auf diese Weise das Verbundmaterial hergestellt. Anschließend erfolgt das Bedrucken des Verbundmaterials bevor Rill- und Falzlinien auf dasselbe aufgebracht werden. Bei diesen Bearbeitungsschritten liegt das Material in der Regel als Rollenware vor. Im nächsten Arbeitsschritt wird das Verpackungsmaterial unter Erhalt eines Zuschnitts ("blank") gestanzt und zu Stapeln geschichtet. Nach diesem Prozessschritt kann das erfindungsgemäße Verfahren sinnvoll angewendet werden. Anschließend entsteht durch Falten des Zuschnitts und Verbinden der seitlichen Schnittkanten (Längsnahtsiegelung) ein oben und unten offener Packungsmantel. Bevorzugt wird das erfindungsgemäße Verfahren an dieser Stelle durchgeführt und erfolgt damit nicht im aseptischen Bereich und sogar außerhalb der Füllmaschine.

Zweckmäßiger Weise ist eine Inkubationszeit für das Entkeimungsmittel vorzusehen. Diese liegt im Bereich von einigen Minuten bis zu mehreren Stunden und reicht aus, die Desinfektion der Kanten über die Transportzeit der Packungsmäntel zur Füllmaschine sicherzustellen. In der Regel verbleibt das Behandlungsmittel dauerhaft im Packungsmantel.

Optional werden mehrere Packungsmäntel vor oder nach der erfindungsgemäßen Behandlung in einer Umverpackung verpackt. Nach dem Transport zur Füllmaschine wird der Packungsmantel aufgefaltet, geformt und auf der Ober- oder Unterseite verschlossen. Nach dem einseitigen Verschließen des Packungsmantels kann das erfindungsgemäße Verfahren ebenfalls angewendet werden. Die Sterilisation der Innenseite des Packungsmantels erfolgt, bevor er im aseptischen Bereich befüllt und verschlossen wird.

Die Behandlung erfolgt inline mit oder offline zu weiteren Behandlungsschritten. Bevorzugt ist das Behandlungsmittel flüssig und/oder hat bei der Behandlung Raumtemperatur. Die Eindringtiefe des Entkeimungsmittels in die Kanten des Verpackungsmaterials beträgt mindestens 1 mm, bevorzugt zwischen 1 und 2 mm. Die Kanten können, abhängig vom Behandlungsmittel, durch dieses ausgeblichen werden, was insbesondere dann der Fall ist, wenn das Verpackungsmaterial aus ungebleichtem Karton besteht oder diesen enthält.

Durch das zusätzliche Behandeln der offenen Schnittkanten mit Entkeimungsmittel und das Eindringen des Entkeimungsmittels in das Verpackungsmaterial werden die offenen Kanten und auf diesen abgelagerte und/oder von diesen aufgenommene Partikel, insbesondere Staub, wirkungsvoll entkeimt und damit das Kontaminationsrisiko reduziert.

Das Aufbringen des Entkeimungsmittels auf die offenen Schnittkanten kann über eine Düse (Sprühen), durch Tauchen der Schnittkanten in ein Bad, über Bürsten, Rollen und/oder Walzen erfolgen.

Erfindungsgemäß sind während des Aufbringens eine Mehrzahl von Packungsmänteln flach gefaltet, bevorzugt in einer geöffneten Umverpackung, zusammengefasst. Ziel dieses Vorgehens ist ein möglichst effizienter Einsatz des Entkeimungsmittels.

Ferner wird bevorzugt, dass die Packungsmäntel während der Behandlung kontinuierlich bewegt werden. Es ist jedoch auch denkbar, dass die Packungsmäntel nicht beweget werden und die Vorrichtung zum Aufbringen des Behandlungsmittels relativ zu den Packungsmänteln bewegt wird. Dem Fachmann sind verschiedene Mittel zum kontinuierlichen Bewegen von Produkten oder Vorrichtungen bekannt. Besonders bevorzugt werden die Packungsmäntel mit einem Förderband oder durch Rollen relativ zu einer ortsgebundenen Düse bewegt.

Eine weitere Lehre der Erfindung sieht vor, dass das verwendete Entkeimungsmittel flüssig ist. Bevorzugt enthält das Entkeimungsmittel Wasserstoffperoxid oder Peressigsäure. Besonders bevorzugt ist es, wenn der Wasserstoffperoxid-Gehalt 35% beträgt, oder, dass der Wasserstoffperoxid-Gehalt nur 2% beträgt und das Entkeimungsmittel anschließend mit einer UV-Strahlung bestrahlt wird. Vorteilhaft ist es ebenfalls, wenn das Behandlungsmittel ein Benetzungsmittel, beispielsweise eine oberflächenaktive Substanz, zum verbesserten Eindringen des Behandlungsmittels in das Verpackungsmaterial enthält. Ein bevorzugtes Mischverhältnis ist:
Behandlungsmittel 1000 : Benetzungsmittel 1

Zusätzlich oder alternativ ist das Behandlungsmittel ein Gemisch, das Wasserstoffperoxid und Alkohol enthält und/oder zusätzlich eine Imprägnierlösung und/oder eine Hydrophobierungslösung. Weiterhin kann das Behandlungsmittel einen Indikator, beispielsweise Farbpigmente, enthalten. Ferner ist bevorzugt, dass das erfindungsgemäße Verfahren eine Reduzierung der Gesamtkoloniezahl bestimmt nach DIN 54379 von mindestens log 2 (∼99%) erreicht.

Gemäß einer weiteren Lehre der Erfindung wird das Behandlungsmittel durch eine Düse aufgebracht.

Gemäß einer weiteren Ausgestaltung der Erfindung ist es vorteilhaft, wenn vor dem Aufbringen des Entkeimungsmittels mindestens die offenen Schnittkanten von Staub befreit werden, insbesondere durch eine Staubabsaugung und/oder durch eine Abstreifeinrichtung. Die Abstreifeinrichtung kann beispielsweise eine rotierende Bürste sein und/oder Borsten aufweisen. Wird vor dem Aufbringen des Entkeimungsmittels bereits lösbarer Staub entfernt, erleichtert dies das Eindringen des Entkeimungsmittels in das Verpackungsmaterial. Außerdem stellen vor dem aseptischen Bereich entfernte Staubkörner kein Kontaminationsrisiko mehr dar. Ferner wird so auch die Verschmutzung der Füllmaschine, insbesondere der Siegelwerkzeuge, reduziert und die Wartungs- und/oder Reinigungsintervalle der Siegelwerkzeuge werden verlängert.

Eine weitere bevorzugte Ausführungsform sieht vor, dass die während der Behandlung entstehenden Dämpfe abgesaugt, insbesondere von einer Absaugung aufgenommen und einer Abluftreinigung und/oder einem Abluftwäscher zugeführt werden. Da von den Dämpfen ein Gesundheitsrisiko ausgehen kann, kann durch eine Absaugung die Belastung für die Umwelt reduziert werden. Hier sind insbesondere die einschlägigen Vorschriften zu beachten.

Gemäß einer weiteren Lehre der Erfindung werden die oberen und/oder unteren offenen Schnittkanten imprägniert. Bevorzugt geschieht das Imprägnieren nach der Behandlung mit dem Entkeimungsmittel. Dem Fachmann sind aus dem Stand der Technik verschiedene Imprägniermittel für das Imprägnieren von Schnittkanten bekannt. Aus der DE 25 22 546 A1 ist für sich ein Verfahren zur Behandlung der Stirnseiten einer Rolle bzw. eines Zuschnitts mit Imprägnierlösung bekannt, um das Eindringen von Flüssigkeit in die offene Kante zu verhindern.

Gemäß einer weiteren Lehre wird der Packungsmantel bestrahlt, insbesondere mit UV-Strahlung, Beta- oder Gamma-Strahlung. Die Bestrahlung erfolgt bevorzugt vor oder nach dem Aufbringen des Entkeimungsmittels bzw. nach der Imprägnierung. Die Bestrahlung wirkt bereits allein keimabtötend, steigert im Falle einer Bestrahlung nach dem Aufbringen des Entkeimungsmittels aber auch zusätzlich dessen abtötende Wirkung.

Nach einer weiteren Ausgestaltung der Erfindung wird unmittelbar nach der Behandlung die Umverpackung, welche eine bestimmte Anzahl von Packungsmänteln enthält, verschlossen. Dies verhindert, dass sich Fremdkörper, insbesondere Staub, neu an den Kanten anlagern und nicht wirksam entkeimt werden und beim Verschließen der Packungsmäntel nach dem Befüllen in der Füllmaschine ein Kontaminationsrisiko darstellen. Hierbei ist es bevorzugt, dass auch die Innenseiten der oberen und/oder unteren Flächen der Umverpackung mit dem Behandlungsmittel behandelt werden. Dies hat den Vorteil, dass nach dem Verschließen eine Re-Kontamination bereits behandelter Flächen reduziert wird. Diese Behandlung kann beispielsweise in der gleichen Vorrichtung erfolgen.

Des Weiteren wird die Aufgabe durch einen Packungsmantel nach dem Oberbegriff von Anspruch 15 dadurch gelöst, dass das Entkeimungsmittel mindestens in den Endbereich der oberen und/oder unteren Schnittkanten eingedrungen ist. Bevorzugt erfolgt das Eindringen des Entkeimungsmittels in einem Bereich von 1 mm bis 2 mm in das Verpackungsmaterial (von der Schnittkante aus gemessen).

Ein Behandeln von Packungsmänteln im Endbereich der oberen und/oder unteren Schnittkanten mit Entkeimungsmittel entkeimt diese und dort abgelagerte Partikel wirkungsvoll, so dass beim Schließen der Packungsmäntel nach dem Befüllen von diesen und auf diesen abgelagertem Staub kein Keimrisiko für den aseptischen Bereich und nachfolgende Packungsmäntel mehr ausgeht.

Insbesondere bei der Behandlung von ungebleichtem Zellstoff kann es durch das eingedrungene Entkeimungsmittel mindestens im Endbereich der oberen und/oder unteren Schnittkanten zu einem Ausbleichen führen, bevorzugt ebenfalls im Bereich von 1 mm bis 2 mm Tiefe.

Das vorbeschriebene Verfahren entkeimt die offenen Schnittkanten, sowie die oberen und/oder unteren Innenseiten der Umverpackung, wirkungsvoll und dauerhaft, so dass die Kanten auch nach einer Zeit von bis zu mehreren Wochen und dem Zurücklegen langer Transportstrecken noch wirksam entkeimt sind.

Die Erfindung wird nachfolgend anhand einer lediglich bevorzugte Ausführungsbeispiele darstellenden Zeichnung näher erläutert.

In der Zeichnung zeigen
- Fig. 1: eine Vorrichtung zum Aufbringen eines Behandlungsmittels auf offene Schnittkanten eines Mantels aus Verpackungsmaterial mit zwei Behandlungsstationen und
- Fig. 2: eine Vorrichtung zum Aufbringen eines Behandlungsmittels auf offene Schnittkanten eines Mantels aus Verpackungsmaterial mit vier Behandlungsstationen.

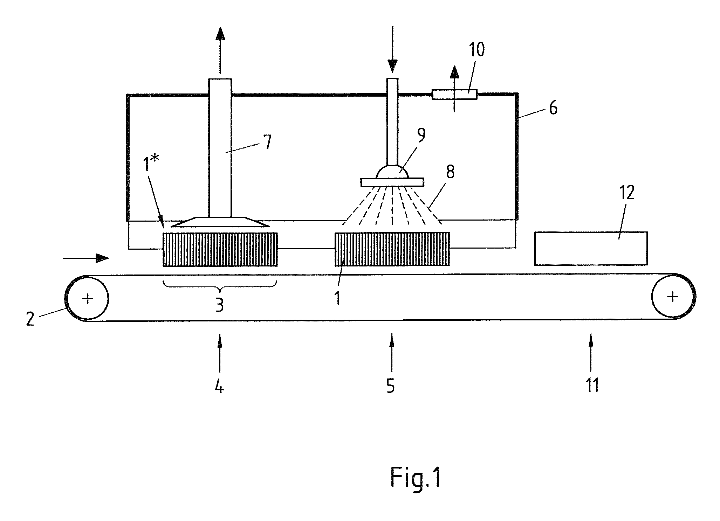

Fig. 1 zeigt ein Ausführungsbeispiel einer Vorrichtung zur Behandlung der oberen Schnittkanten 1* von Packungsmänteln 1. Die Packungsmäntel 1 befinden sich als Gruppe aus mehreren Packungsmänteln 1 zusammengefasst in einer offenen Umverpackung 3. Es ist ein Förderband 2 gezeigt, auf dem die offene Umverpackung 3 zwei Behandlungsstationen 4 und 5 durchläuft. Beide Behandlungsstationen sind durch eine gemeinsame Einhausung 6 von der Umgebung getrennt.

In der ersten (optionalen) Behandlungsstation 4 werden Partikel, insbesondere Staub, mit Hilfe einer Staubabsaugung 7 mindestens von den oberen Kanten der Packungsmäntel 1 abgesaugt. In der nächsten Behandlungsstation 5 wird ein ein Entkeimungsmittel enthaltendes Behandlungsmittel 8 durch eine Düse 9 mindestens auf die Kanten der Packungsmäntel 1 aufgetragen und kann in diese eindringen.

Das Behandlungsmittel 8 kann zusätzlich ein Benetzungsmittel enthalten. Über der Behandlungsstation 5 kann eine weitere Absaugung 10 zum Absaugen von bei der Behandlung auftretenden Dämpfen vorgesehen sein. Danach verlassen die Packungsmäntel 1 in der offenen Umverpackung die Einhausung 6 wonach in einer Packstation 11 die Umverpackung verschlossen wird. In der verschlossenen Umverpackung 12 erfolgt der Weitertransport der Packungsmäntel 1.

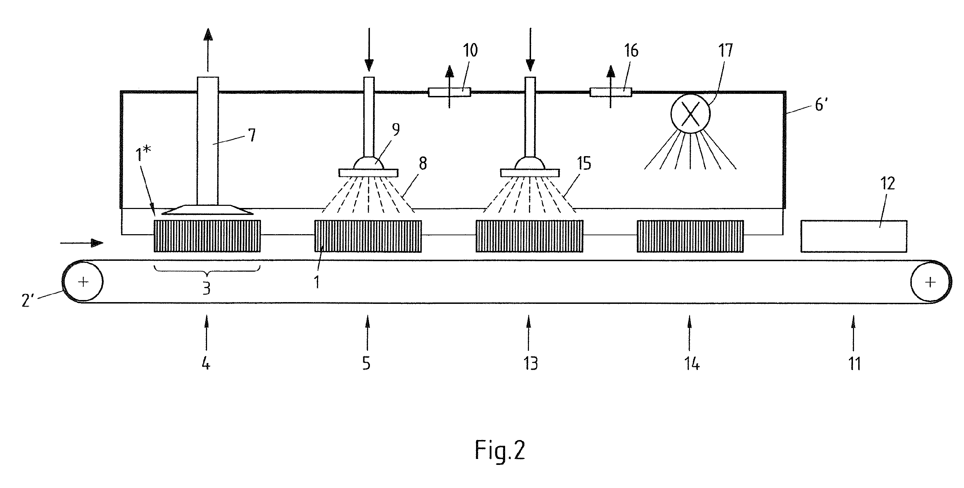

Fig. 2 zeigt ein weiteres Ausführungsbeispiel. An die beiden bereits in Fig. 1 gezeigten Behandlungsstationen 4 und 5 schließen sich eine dritte Behandlungsstation 13 und eine vierte Behandlungsstation 14 an, bevor die offene Umverpackung 3 in einer Packstation 11 verschlossen wird. In diesem Ausführungsbeispiel sind alle vier Behandlungsstationen 4, 5, 13, 14 über ein Förderband 2' durch eine Einhausung 6' von der Umgebung getrennt.

Die dritte Behandlungsstation 13 ist optional und imprägniert mindestens die Kanten 1* der Packungsmäntel 1 mit einem Imprägniermittel 15. Über dieser Behandlungsstation 13 kann ebenfalls eine Absaugung 16 zum Absaugen von bei der Behandlung auftretenden Dämpfen vorgesehen sein. Diese kann mit der Absaugung 10 von der zweiten Behandlungsstation 5 verbunden sein. Die Absaugungen 10, 16 können mit einer nicht gezeigten Abluftreinigungseinrichtung oder einem nicht gezeigten Abluftwäscher verbunden sein.

In der vierten Behandlungsstation 14 werden mindestens die offenen oberen Kanten der Packungsmäntel 1 von einer Strahlenquelle 17 mit UV- und/oder Beta- und/oder GammaStrahlen bestrahlt. Danach verlassen die behandelten Packungsmäntel 1 in der offenen Umverpackung 3 die Einhausung 6', werden verschlossen und die geschlossenen Umverpackungen 12 können zum Abfüllort transportiert werden.

Es versteht sich, dass die gezeigten Ausführungsbeispiele nur Möglichkeiten zeigen, das erfindungsgemäße Verfahren auszugestalten, ohne eine Einschränkung auf die gezeigten Vorrichtungen darzustellen.

## Patentansprüche

1. Verfahren zur Behandlung von oben und unten offenen Schnittkanten eines Rohlings ("Packungsmantel") aus Verpackungsmaterial, insbesondere Papier/Kunststoff-Verbundmaterial, durch Aufbringen eines Behandlungsmittels,
**dadurch gekennzeichnet, dass** das Behandlungsmittel (8) mindestens ein Entkeimungsmittel enthält, dass während des Aufbringens eine Mehrzahl von Packungsmänteln (1) flach gefaltet zusammengefasst sind und dass das Entkeimungsmittel nach dem Aufbringen auf den Schnittkanten (1*) verbleibt und in das Verpackungsmaterial eindringt.

2. Verfahren nach Anspruch 1,
**dadurch gekennzeichnet, dass** die Packungsmäntel (1) während der Behandlung kontinuierlich bewegt werden.

3. Verfahren nach Anspruch 1,
**dadurch gekennzeichnet, dass** eine Behandlungsvorrichtung relativ zu den Packungsmänteln (1) bewegt wird.

4. Verfahren nach einem der Ansprüche 1 bis 3,
**dadurch gekennzeichnet, dass** das Entkeimungsmittel flüssig ist.

5. Verfahren nach einem der Ansprüche 1 bis 4,
**dadurch gekennzeichnet, dass** das Entkeimungsmittel Raumtemperatur besitzt.

6. Verfahren nach einem der Ansprüche 1 bis 5,
**dadurch gekennzeichnet, dass** das Entkeimungsmittel Wasserstoffperoxid oder Peressigsäure enthält.

7. Verfahren nach Anspruch 5 oder 6,
**dadurch gekennzeichnet, dass** das Entkeimungsmittel zusätzlich ein Benetzungsmittel enthält.

8. Verfahren nach einem der Ansprüche 1 bis 7,
**dadurch gekennzeichnet, dass** das Behandlungsmittel durch eine Düse (9) mindestens auf die Schnittkanten (1*) aufgebracht wird.

9. Verfahren nach einem der Ansprüche 1 bis 8,
**dadurch gekennzeichnet, dass** vor dem Aufbringen des Entkeimungsmittel mindestens die offenen Schnittkanten (1*) von Staub befreit werden, insbesondere durch eine Staubabsaugung (7) und/oder durch eine Abstreifeinrichtung.

10. Verfahren nach einem der Ansprüche 1 bis 9,
**dadurch gekennzeichnet, dass** die während der Behandlung entstehenden Dämpfe abgesaugt, insbesondere von einer Absaugung (16) aufgenommen und einer Abluftreinigung und/oder einem Abluftwäscher zugeführt werden.

11. Verfahren nach einem der Ansprüche 1 bis 10,
**dadurch gekennzeichnet, dass** die oberen und/oder unteren offenen Schnittkanten (1*) der Packungsmäntel (1) imprägniert werden.

12. Verfahren nach einem der Ansprüche 1 bis 11,
**dadurch gekennzeichnet, dass** der Packungsmantel (1) bestrahlt wird, insbesondere mit UV-Strahlung, Beta- oder Gamma-Strahlung.

13. Verfahren nach einem der Ansprüche 1 bis 12,
**dadurch gekennzeichnet, dass** eine bestimmte Anzahl von Packungsmänteln (1) in einer Umverpackung (3) zusammengefasst sind und das Behandlungsmittel mindestens auf die Schnittkanten (1*) aufgebracht wird.

14. Verfahren nach Anspruch 13,
**dadurch gekennzeichnet, dass** unmittelbar nach der Behandlung die offene Umverpackung (3) zu einer geschlossenen Umverpackung (12) verschlossen wird.

15. Packungsmantel, hergestellt nach einem Verfahren nach einem der Ansprüche 1 bis 14,
**dadurch gekennzeichnet, dass** das Entkeimungsmittel mindestens in den Endbereich der behandelten Schnittkanten (1*) eingedrungen ist.

## Claims

1. Method for treating the top and bottom open cut edges of a blank ("carton sleeve") made of packaging material, in particular paper/plastic composite material, by applying a treatment agent,
**characterised in that**
said treatment agent (8) contains at least one disinfectant, **in that**, during the application, a plurality of carton sleeves (1) are folded flat, and **in that** after the application, the disinfectant remains on the cut edges (1*) and penetrates the packaging material.

2. Method according to claim 1,
**characterised in that**
the carton sleeves (1) are moved continuously during treatment.

3. Method according to claim 1,
**characterised in that**
a treatment device is moved relatively to the carton sleeves (1).

4. Method according to any one of claims 1 to 3,
**characterised in that**
the disinfectant is a liquid.

5. Method according to any one of claims 1 to 4,
**characterised in that**
the disinfectant is at room temperature.

6. Method according to any one of claims 1 to 5,
**characterised in that**
the disinfectant contains hydrogen peroxide or peracetic acid.

7. Method according to claim 5 or 6,
**characterised in that**
the disinfectant additionally contains a wetting agent.

8. Method according to any one of claims 1 to 7,
**characterised in that**
the treatment agent is applied through a nozzle (9) at least to the cut edges (1*).

9. Method according to any one of claims 1 to 8,
**characterised in that**,
before application of the disinfectant, dust is removed at least from the open cut edges (1*), in particular by a dust extractor (7) and/or by a wiper unit.

10. Method according to any one of claims 1 to 9,
**characterised in that**
the vapours generated during the treatment are extracted, in particular captured by an extractor (16) and supplied to an exhaust air purifier and/or an exhaust air scrubber.

11. Method according to any one of claims 1 to 10,
**characterised in that**
the upper and/or lower open cut edges (1*) of the carton sleeves (1) are impregnated.

12. Method according to any one of claims 1 to 11,
**characterised in that**
the carton sleeve (1) is irradiated, in particular with UV radiation, beta or gamma radiation.

13. Method according to any one of claims 1 to 12,
**characterised in that**
a certain number of carton sleeves (1) are combined in an outer package (3) and the treatment agent is applied at least to the cut edges (1*).

14. Method according to claim 13,
**characterised in that**
immediately after the treatment, the open outer package (3) is sealed as a closed outer package (12).

15. Carton sleeve, manufactured by a method according to any one of claims 1 to 14, **characterised in that**
the disinfectant has penetrated at least the end region of the treated cut edges (1*).

## Revendications

1. Procédé de traitement d'arêtes de coupe d'une ébauche ("manchon d'emballage"), ouvertes sur le haut et sur le bas, se composant d'un matériau d'emballage, en particulier d'un matériau composite à base de papier et de matière plastique, ledit traitement étant effectué par application d'un moyen de traitement,
**caractérisé en ce que** le moyen de traitement (8) contient au moins un agent désinfectant, **en ce que**, au cours de l'application, des manchons d'emballage (1), formant une pluralité d'éléments, sont regroupés en étant pliés à plat, et **en ce que** l'agent désinfectant, après l'application, reste sur les arêtes de coupe (1*) et pénètre dans le matériau d'emballage.

2. Procédé selon la revendication 1,
**caractérisé en ce que** les manchons d'emballage (1) sont déplacés en continu au cours du traitement.

3. Procédé selon la revendication 1,
**caractérisé en ce qu'**un dispositif de traitement est déplacé par rapport aux manchons d'emballage (1).

4. Procédé selon l'une quelconque des revendications 1 à 3,
**caractérisé en ce que** l'agent désinfectant est liquide.

5. Procédé selon l'une quelconque des revendications 1 à 4,
**caractérisé en ce que** l'agent désinfectant a une température ambiante.

6. Procédé selon l'une quelconque des revendications 1 à 5,
**caractérisé en ce que** l'agent désinfectant contient du peroxyde d'hydrogène ou de l'acide peracétique.

7. Procédé selon la revendication 5 ou 6,
**caractérisé en ce que** l'agent désinfectant contient en outre un agent mouillant.

8. Procédé selon l'une quelconque des revendications 1 à 7,
**caractérisé en ce que** l'agent de traitement est appliqué par une buse (9) au moins sur les arêtes de coupe (1*).

9. Procédé selon l'une quelconque des revendications 1 à 8,
**caractérisé en ce que**, avant l'application de l'agent désinfectant, au moins les arêtes de coupe ouvertes (1*) sont dépoussiérées, en particulier en procédant à une aspiration (7) de la poussière et/ou en utilisant un dispositif de raclage.

10. Procédé selon l'une quelconque des revendications 1 à 9,
**caractérisé en ce que** les vapeurs se produisant au cours du traitement sont aspirées, en particulier captées par un dispositif d'aspiration (16) et fournies à un dispositif d'épuration de l'air vicié et/ou à un laveur de l'air vicié.

11. Procédé selon l'une quelconque des revendications 1 à 10,
**caractérisé en ce que** les arêtes de coupe (1*) des manchons d'emballage (1), ouvertes sur le haut et/ou sur le bas, sont imprégnées.

12. Procédé selon l'une quelconque des revendications 1 à 11,
**caractérisé en ce que** le manchon d'emballage (1) est irradié, en particulier par rayonnement ultraviolet, par rayonnement bêta ou gamma.

13. Procédé selon l'une quelconque des revendications 1 à 12,
**caractérisé en ce qu'**un nombre déterminé de manchons d'emballage (1) est regroupé dans un suremballage enveloppant (3), et le moyen de traitement est appliqué au moins sur les arêtes de coupe (1*).

14. Procédé selon la revendication 13,
**caractérisé en ce que**, immédiatement après le traitement, le suremballage enveloppant ouvert (3) est fermé, pour former un suremballage enveloppant fermé (12).

15. Manchon d'emballage fabriqué d'après un procédé selon l'une quelconque des revendications 1 à 14,
**caractérisé en ce que** l'agent désinfectant a pénétré au moins dans la zone d'extrémité des arêtes de coupe traitées (1*).
